(19) Europäisches Patentamt
European Patent Office
Office européen des brevets

(11) **EP 4 350 702 A1**

(12) **EUROPEAN PATENT APPLICATION**

(43) Date of publication:
**10.04.2024 Bulletin 2024/15**

(21) Application number: **22210193.3**

(22) Date of filing: **29.11.2022**

(51) International Patent Classification (IPC):
**G16H 10/40** (2018.01)   **G01N 33/487** (2006.01)
**G01N 35/00** (2006.01)   **G16H 40/67** (2018.01)
**H04M 1/72412** (2021.01)   **H04W 4/38** (2018.01)
**H04W 4/80** (2018.01)   **H04B 5/00** (2024.01)
**H04B 5/02** (2006.01)   **B01L 3/00** (2006.01)

(52) Cooperative Patent Classification (CPC):
**G16H 10/40; G01N 33/48792; G01N 35/00871;
G16H 40/67; H04W 4/38; H04M 1/72412;
H04W 4/80**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.10.2022 KR 20220128344**

(71) Applicant: **1Drop Inc.
Gyeonggi-do 13217 (KR)**

(72) Inventors:
• **JANG, Sung Woo
Seongnam 13562 (KR)**
• **RHEE, Joo Won
Seongnam 13562 (KR)**

(74) Representative: **advotec.
Patent- und Rechtsanwaltspartnerschaft
Tappe mbB
Widenmayerstraße 4
80538 München (DE)**

Remarks:
Claim 16 to19 are deemed to be abandoned due to non-payment of the claims fee (Rule 45(3) EPC).

(54) **METHOD AND APPARATUS FOR EFFICIENTLY TRANSMITTING NUCLEIC ACID AMPLIFICATION RESULT DATA MADE BY MEASURING INSTRUMENT**

(57)   The present specification provides a method and apparatus capable of efficiently transmitting data related to test results made by a plurality of measuring instruments. The method of transmitting measurement result data according to the present specification may transmit identification information on a measuring instrument in which a positive reaction is detected to a server. As a result, data made by the plurality of measuring instruments may be transmitted with a small capacity, and thus communication resources can be saved.

Fig. 5

EP 4 350 702 A1

**Description**

**1. Field of the Invention**

The present invention relates to a method of controlling a communication terminal, and more particularly, to a communication terminal for transmitting data measured by an in vitro diagnostic medical device.

**2. Discussion of Related Art**

The contents described in this section merely provide background information for embodiments disclosed in the present specification, and do not constitute the related art.

Since Coronavirus Disease-19 (COVID-19) was first reported in Wuhan, China in December 2019, the number of confirmed cases and deaths worldwide continues to rise. Covid-19 was initially known as a respiratory infectious disease with unknown cause. However, the World Health Organization (WHO) confirmed that Covid-19 was a new type of coronavirus, and as Covid-19 spreads not only in Korea, but also in Europe, the United States, etc., the WHO has declared COVID-19 a pandemic, the highest-risk level among the infectious disease alert levels on March 11, 2020 and warned of the seriousness of Covid-19.

For the diagnosis of COVID-19, regardless of the clinical aspect, a test standard for diagnosis is a method of separating a virus from a specimen or detecting a specific viral gene. The genetic test used here is real-time reverse transcription polymerase chain reaction (real-time RT-PCR). This method has an advantage of being an accurate test with close to 100% sensitivity and specificity, but has a disadvantage that it requires professional manpower to collect a sample and takes a long time to derive a test result.

A measuring instrument using loop-mediated isothermal amplification (LAMP), which has a relatively short measurement time compared to the PCR test, is attracting attention as a device for on-site diagnosis.

[Related Art Document]

[Patent Document]

(Patent Document 1) Korean Patent Publication No. 10-2229225

SUMMARY OF THE INVENTION

The present disclosure provides a method and apparatus capable of efficiently transmitting data related to test results made by a plurality of measuring instruments.

The present disclosure is not limited to the above-described objects, and other objects that are not mentioned may be obviously understood by those skilled in the art from the following description.

According to an aspect of the present invention, a method of transmitting measurement result data may include operations of (a) storing, by a processor, identification information for a plurality of measuring instruments connected through short-range wireless communication; (b) determining, by the processor, whether a nucleic acid to be detected is included in a sample being measured by each measuring instrument (hereinafter, "positive or negative") using measured values output from each measuring instrument; and (c) transmitting, by the processor, identification information on a measuring instrument having a sample determined to be positive among the plurality of measuring instruments to a server.

The operation (c) may include: transmitting, by the processor, the identification information on the measuring instrument having a sample determined to be positive as first group information, and transmitting, by the processor, identification information on a measuring instrument having a sample determined to be negative as second group information.

The operation (a) may include transmitting, by the processor, the identification information for the plurality of measuring instruments to the server, and the operation (c) may include transmitting, by the processor, the identification information on the measuring instrument having the sample determined to be positive as first group information.

The operation (a) may include further storing an order in which the identification information on each measuring instrument is written as index information on each measuring instrument when the processor transmits the identification information for the plurality of measuring instruments to the server, and the operation (c) may include transmitting, by the processor, the index information on the measuring instrument having the sample determined to be positive as first group information.

The operation (b) may include further storing, by the processor, a measured value (hereinafter, 'time series data') output at each preset time in the measurement process in each measuring instrument, and the operation (c) may include further transmitting, by the processor, the time series data of the measuring instrument having the sample determined to be positive to the server.

The operation (c) may include editing, by the processor, the time series data using a difference value between a preset reference value and each measured value and transmitting the edited time series data to the server.

The operation (c) may include editing, by the processor, the time series data using a difference value between a minimum value in the time series data and each measured value and transmitting the edited time series data to the server.

The operation (c) may include deriving, by the processor, a trend line of the time series data and transmitting the derived time series data to the server.

The operation (c) may include calculating, by the processor, error values between each measured value included in the time series data and the derived trend line and transmitting a coefficient value of the derived trend line to the server when the number of the error values exceeding a preset error range is less than or equal to a preset reference number.

A non-transitory computer readable recording medium recording a computer program for performing operations of the measurement result data transmitting method in a computer and recorded on a computer-readable recording medium.

According to another aspect of the present invention, an apparatus for transmitting measurement result data may include: a short-range wireless communication unit configured to transmit and receive data to and from a plurality of measuring instruments; a storage unit configured to store data received through the short-range wireless communication unit; a server communication unit configured to transmit and receive the data to and from a server; and a control unit configured to control a server communication unit to store identification information for the plurality of measuring instruments connected through the short-range wireless communication unit in the storage unit, use measured values output from each measuring instrument to determine whether a nucleic acid to be detected is included in a sample being measured by each measuring instrument (hereinafter referred to as "positive or negative"), and transmit identification information on the measuring instrument having a sample determined to be positive among the plurality of measuring instruments to the server.

The control unit may transmit the identification information on the measuring instrument having the sample determined to be positive as first group information, and allow the processor to transmit identification information on a measuring instrument having a sample determined to be negative as second group information.

The control unit may transmit the identification information for the plurality of measuring instruments to the server, and transmit the identification information on the measuring instrument having the sample determined to be positive as first group information.

When the processor transmits the identification information for the a plurality of measuring instruments to the server, the control unit may further store an order, in which the identification information on each measuring instrument is written, as index information on each measuring instrument, and transmit the index information on the measuring instrument having the sample determined to be positive as the first group information.

The control unit may allow the processor to further store the measured value (hereinafter, "time series data") output at each preset time in the measurement process in each measuring instrument, and further store the time series data of the measuring instrument having the sample determined to be positive to the server.

The control unit may edit the time series data using a difference value between a preset reference value and each measured value and transmit the edited time series data to the server.

The control unit may edit the time series data using a difference value between a minimum value in the time series data and each measured value and transmit the edited time series data to the server.

The control unit may derive a trend line of the time series data and transmit a coefficient value of the derived trend line to the server.

The control unit may calculate error values between each measured value included in the time series data and the derived trend line and transmit the coefficient value of the derived trend line to the server when the number of the error values exceeding a preset error range is less than or equal to a preset reference number.

Other specific details of the present invention are included in the detailed description and the accompanying drawings.

BRIEF DESCRIPTION OF THE DRAWINGS

FIG 1 is a reference diagram for helping understanding of a diagnosis system according to the present specification.

FIG 2 is a block diagram schematically illustrating a configuration of a measuring instrument according to the present specification.

FIGS. 3A and 3B are exemplary views in which a color of a tube changes.

FIG 4 is an exemplary table of interpretation of results according to the color of the tube.

FIG 5 is a schematic configuration diagram of a communication terminal connected to a plurality of measuring instruments and a server.

FIG 6 is a partial example of data for a plurality of measurement results.

FIG 7 is a block diagram schematically illustrating a configuration of an apparatus for transmitting measurement result data according to the present specification.

FIG 8 is a schematic flowchart of a method of transmitting measurement result data according to the present specification.

FIG 9 is a diagram illustrating data on results of a Korean coronavirus test from February to November 2021.

FIG 10 is a flowchart of a method of transmitting measurement result data according to a first embodiment of the present specification.

FIG 11 is an example of data transmitted according to the first embodiment of the present specification.

FIG 12 is a flowchart of a method of transmitting measurement result data according to a second embodiment of the present specification.

FIG 13 is an example of data transmitted according to the second embodiment of the present specification.

FIG 14 is a flowchart of a method of transmitting measurement result data according to a third embodiment of the present specification.

FIG 15 is an example of data transmitted according to the third embodiment of the present specification.

FIG 16 is an example of a change in absorbance of a control tube and a test tube over time for a sample determined to be negative.

FIG 17 is an example of a change in absorbance of a control tube and a test tube over time for a sample determined to be positive.

FIG 18 is a flowchart of a method of transmitting measurement result data according to a fourth embodiment of the present specification.

FIG 19 is an example of time series data.

FIG 20 is a flowchart of a method of transmitting measurement result data according to a fifth embodiment of the present specification.

FIG 21 is an example of editing time series data using a preset reference value.

FIG 22 is a flowchart of a method of transmitting measurement result data according to a sixth embodiment of the present specification.

FIG 23 is an example of editing time series data using a minimum value in time series data.

FIG 24 is a flowchart of a method of transmitting measurement result data according to a seventh embodiment of the present specification.

FIG 25 is an exemplary diagram of a trend line of time series data.

## DETAILED DESCRIPTION OF EXEMPLARY EMBODIMENTS

Various advantages and features of the present specification and methods accomplishing them will become apparent from the following description of embodiments with reference to the accompanying drawings. However, the present specification is not limited to embodiments to be described below, but may be implemented in various different forms, these embodiments will be provided only in order to make the present specification complete and allow a person with ordinary skill in the art (hereinafter, those skilled in the art) to which the present specification pertains to completely recognize the scope of the present specification, and the scope of the present specification will be defined by the scope of the claims.

Terms used in the present specification are for describing embodiments rather than limiting the scope of the present specification. Unless otherwise stated, a singular form includes a plural form in the present specification. Throughout the present specification, the term "comprise" and/or "comprising" will be understood to imply the inclusion of stated constituents but not the exclusion of any other constituents.

Like reference numerals refer to like components throughout the specification and "and/or" includes each of the components mentioned and includes all combinations thereof. Although "first," "second," and the like are used to describe various components, it goes without saying that these components are not limited by these terms. These terms are used only to distinguish one component from other components. Therefore, it goes without saying that a first component described below may be a second component within the technical scope of the present disclosure.

Unless defined otherwise, all terms (including technical and scientific terms) used in the present specification have the same meaning as meanings commonly understood by those skilled in the art to which the present specification pertains. In addition, terms defined in commonly used dictionary are not ideally or excessively interpreted unless explicitly defined otherwise.

Hereinafter, embodiments of the present invention will be described in detail with reference to the accompanying drawings.

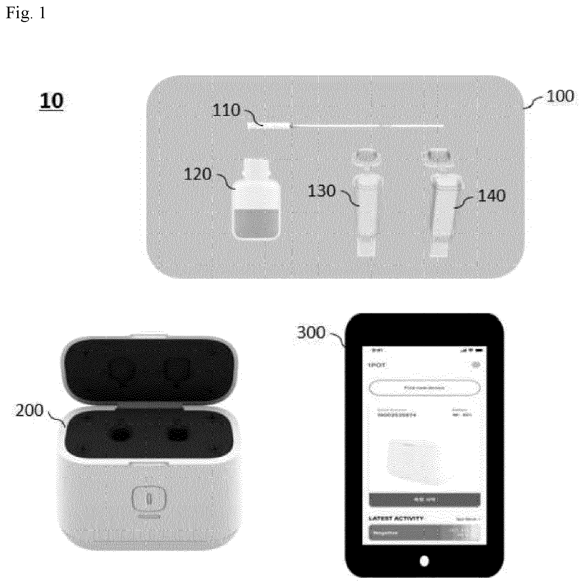

FIG 1 is a reference diagram for helping understanding of a diagnosis system according to the present specification.

Referring to FIG 1, a diagnostic system 10 according to the present specification may include a test device 200 and a communication terminal 300.

The test device 200 is a device capable of performing a test on a specimen collected from a respiratory system. The test device 200 may be a molecular diagnostic device or an antigen diagnostic device. In the present specification, as an example of the test device 200, a measuring instrument 200 capable of performing a test on a specimen through a molecular diagnostic method is presented. The molecular diagnostic method refers to a method of checking whether or not a disease is infected by extracting DNA or RNA from a specimen (saliva, blood, etc.) of a person suspected of being infected with a virus or bacteria through gene amplification technology and amplifying the extracted DNA or RNA. Depending on the gene amplification method, there are various methods such as polymerase chain reaction (PCR), reverse transcription polymerase chain reaction (RT-PCR), and loop-mediated isothermal amplification (LAMP). In the present specification, an example of the measuring instrument 200 that measures an infection by qualitatively quantifying the novel coronavirus (SARS-CoV-2) genes (S gene and N gene) by LAMP with a sample collected from the nasopharynx or nasal cavity of a patient suspected of having a novel coronavirus (SARS-CoV-2) infection will be described. However, in the above example, the test device is not limited to the molecular diagnosis or the LAMP method.

In order to perform the test on the specimen using the measuring instrument 200 according to the present specification, a test kit may be required. The test kit refers to tools, reagents, and consumables required for testing, and may be sealed and provided in a pouch 100.

Referring to FIG 1, a swab 110, a sample tube 120, a test tube 130, and a control tube 140 may be identified. The swab 110, the sample tube 120, the test tube 130, and the control tube 140 may be manufactured for one-time use by one subject. Therefore, the swab 110, the sample tube 120, the test tube 130, and the control tube 140 may be provided as new components for each test for additional and repeated tests. In addition, the swab 110, the sample tube 120, the test tube 130, and the control tube 140 may be manufactured in a sterile state, and distributed while being sealed in the pouch 100.

The swab 110 is a tool (sampling tool) for a subject to collect a specimen. A length and a thickness of the swab 110 may have values suitable for collecting a sample.

The sample tube 120 may include a solution for nucleic acid extraction. Specifically, the nucleic acid extraction solution may include a buffer solution, a nonionic surfactant, a kosmotropic salt, and an indicator.

In the nucleic acid extraction solution, the buffer solution may be a Tris-buffer solution. Specifically, the buffer solution may be Tris-HCl of pH 8 to pH 9, or pH 8.3 to pH 8.6. A concentration of the buffer solution may be in the range of 1.0 mM to 2 mM.

In the nucleic acid extraction solution, the nonionic surfactant may be a Tween type surfactant. Specifically, the nonionic surfactant may be Tween 20. The concentration of the nonionic surfactant may be in the range of 0.05 vol% to 0.5 vol% or 0.07 vol% to 0.2 vol%.

In the nucleic acid extraction solution, the kosmotropic salt may include at least one selected from the group consisting of ammonium sulfate, potassium chloride, and magnesium sulfate. Specifically, the kosmotropic salt may include all of ammonium sulfate, potassium chloride, and magnesium sulfate. In this case, the concentration of the ammonium sulfate may be in the range of 5 mM to 20 mM or 7.5 mM to 15 mM. In addition, the concentration of the potassium chloride may be in the range of 20 mM to 100 mM or 40 mM to 60 mM. Furthermore, the concentration of the magnesium sulfate may be in the range of 3 mM to 10 mM or 4 mM to 8 mM.

In the nucleic acid extraction solution, the indicator may include at least one selected from the group consisting of Cresol RED and Phenol RED, but is not limited thereto, and an appropriate indicator may be used according to the purpose.

In addition, the nucleic acid extraction solution may further include an additive such as guanidine. When the nucleic acid extraction solution further contains guanidine, the concentration thereof may be in the range of 10 mM to 80 mM or 30 mM to 50 mM.

The test tube 130 and the control tube 140 may include primers for the LAMP method. The primer included in the test tube 130 and the primer included in the control tube 140 are different primers. The primer included in the test tube 130 is a primer capable of amplifying a nucleic acid of a virus to be confirmed for infection, and the primer included in the control tube 140 is a primer capable of amplifying a nucleic acid that may be collected from a general person.

In addition, the test tube 130 and the control tube 140 may further include 0.1 to 1 vM Primer mix, 32 KU/T Bst polymerase, 12.5 mU/T RNase inhibitor, 20 to 200 uM dNTPs, 20 vg/T BSA, 1.2 vg/T Trehalose, 1.6 vg sample buffer, etc.

The measuring instrument 200 may communicate with the communication terminal 300 and may perform a test on a specimen. The measuring instrument 200 may have a structure capable of opening and closing a lid, and a space in which the test tube 130 and the control tube 140 may be housed when the lid is opened. According to the embodiment of the present specification, the test tube 130 and the control tube 140 may have different shapes (e.g., a cylindrical shape and a rectangular cylindrical shape) to be easily distinguished, and the inside of the measuring instrument 200 may have a space to correspond to the shapes of the test tube 130 and the control tube 140.

Meanwhile, the measuring instrument 200 may perform a test for the specimen using LAMP, and may include a configuration required for LAMP.

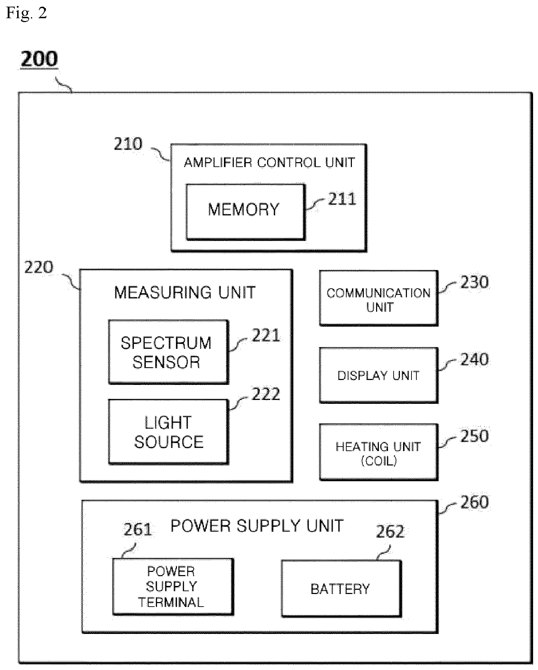

FIG 2 is a block diagram schematically illustrating a configuration of a measuring instrument according to the present specification.

Referring to FIG 2, the measuring instrument 200 according to the present specification may include a measuring instrument control unit 210, a measuring unit 220, a communication unit 230, a display unit 240, a heating unit 250, and a power supply unit 260.

The measuring instrument control unit 210 may control a process necessary for performing a test on a specimen using ring-mediated isothermal amplification (LAMP). When the measuring instrument control unit 210 is able to output a signal for controlling each component included in the measuring instrument 200, the measuring instrument control unit 210 receives a signal from each component and performs processing such as necessary calculations, storage, processing, etc.

The measuring unit 220 may serve to measure the test tube 130 and the control tube 140 according to a procedure for performing a test on a sample. As will be described in more detail later, colors of the solution contained in the test tube 130 and the control tube 140 may change as the nucleic acid is amplified depending on the presence or absence of the nucleic acid. The measuring unit 220 may measure the colors of the test tube 130 and the control tube 140, and output the measured colors as a signal to the control unit 210. To this end, the measuring unit 220 may include a spectrum sensor 221 and/or a light source 222.

The communication unit 230 may serve to transmit/receive data between the measuring instrument 200 and the communication terminal 300. The communication unit 230 may perform wired communication and/or wireless communication, and may transmit and receive data according to a preset communication protocol. Preferably, the communication unit 230 may be a short-range communication module for short-range communication. The short-range communication module may support short-range communication using at least one of Bluetooth™, radio frequency identification (RFID), infrared data association (IrDA), ultra wideband (UWB), ZigBee, near field communication (NFC), wireless-fidelity (Wi-Fi), Wi-Fi Direct, and wireless universal serial bus (wireless USB) technologies. The display unit 240 may serve to display state information and operation information of the measuring instrument 200. The display unit 240 may be two LEDs installed on an outer front surface of the measuring instrument 200. A first LED may display a communication connection state between the measuring instrument 200 and the communication terminal 300, and a second LED may display that the measuring instrument 200 is in a charging state and a normal operating state.

The heating unit 250 may serve to heat the test tube 130 and the control tube 140. The heating unit 250 may be configured as a coil to receive power from the power supply unit 260 by the control signal of the measuring instrument control unit 210 and convert the received power into thermal energy. The coil may have a length and resistance to heat the test tube 130 and the control tube 140 to a preset temperature. According to an embodiment of the present specification, the heating unit 250 may further include a temperature sensor (not illustrated). The measuring instrument control unit 210 may output a signal for controlling the operation of the heating unit 250 according to a temperature signal output from the temperature sensor.

The power supply unit 260 may serve to supply power required for the operation of the components included in the measuring instrument 200 according to the present specification. To this end, the power supply unit 260 may include a power supply terminal 261 and a battery 262. The battery 262 may be both a primary battery and a secondary battery, and preferably, the battery 262 is a secondary battery. The power supply terminal 261 is an interface for connection with an external power supply source (e.g., a commercial power grid, a rechargeable battery, etc.), and may be configured as, for example, a universal serial bus (USB) input/output terminal. The power supply unit 260 may charge the battery 262 with power applied through the power supply terminal 261. The power charged in the battery 262 or power directly received from the power supply terminal 261 may be used to perform a test on a specimen.

The communication terminal 300 according to the present disclosure may be a communication terminal that can transmit/receive data, and may include, for example, a smart phone, a laptop computer, a digital broadcasting terminal, a personal digital assistant (PDA), a portable multimedia player (PMP), a navigation device, a slate PC, a tablet PC, an ultrabook, a wearable device (e.g., smartwatch, smart glasses, head mounted display), or the like. On the communication terminal 300 according to the present specification, an application necessary for performing a test on a specimen may be installed.

The communication terminal 300 may also include a communication unit 230 of the measuring instrument 200 and a communication unit for transmitting/receiving data. In this case, a communication protocol included in the communication terminal 300 is the same as the communication protocol of the communication unit 230 in the measuring instrument 200. In the present specification, for example, it will be described that data communication is possible between the measuring instrument 200 and the communication terminal 300 using a short-range communication module that supports Bluetooth™. Meanwhile, the communication terminal 300 may transmit/receive data between other communication terminals and/or a wireless communication system and/or an external server in addition to the measuring instrument 200. The communication unit of the communication terminal 300 may further include a mobile communication module, a wireless Internet module, or a short-range communication module.

A method of diagnosing a virus infection using the measuring instrument 200 according to the present specification will be described. In this case, it is assumed that the battery included in the measuring instrument 200 is fully charged, and the communication terminal 300 has an application program necessary for performing a test on a specimen installed thereon.

First, a power supply may be turned on by pressing a (power supply) button located on the front of the measuring instrument 200. By executing the application installed on the communication terminal 300, the measuring instrument 200 and the communication terminal 300 may be connected to each other.

Next, a subject may collect a sample by pushing the swab 110 up to the nasopharynx inside the nose. After the sample collection is completed, the swab 110 may be put in the sample tube 120 and mixed by shaking sufficiently about 20 to 30 times.

Next, a preset amount (e.g., 50 µl) of specimen mixed by sufficiently shaking the sample tube 120 may be put into the test tube 130 and the control tube 140. The test tube 130 and the control tube 140 are each mounted in the measuring instrument 200, and the lid of the measuring instrument 200 is closed. When the lid of the measuring instrument 200 is closed, driving may be started at a preset temperature for a preset time. In this case, the remaining time among the total time may be displayed on a display screen of the communication terminal 300.

When the preset time has elapsed, the screen of the communication terminal 300 is switched to a driving completion screen, and the reading result may be displayed on the screen. In this case, the measuring unit 220 of the measuring instrument 200 may output a signal for a change in color of the test tube 130 and the control tube 140. When the signal output from the measuring unit 220 is able to be stored in a memory 211 of the measuring instrument control unit 210, the measuring instrument control unit 210 may transmit the data for the signal stored in the memory 211 to the communication terminal 300 through the communication unit 230.

The communication terminal 300 may determine whether an infection has occurred using the data on the change in color of the test tube 130 and the control tube 140 and display the determination result on the screen.

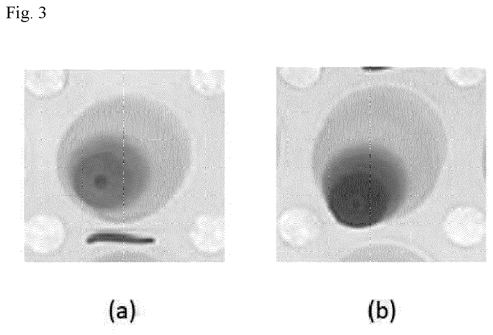

FIGS. 3A and 3B are exemplary views in which a color of a tube changes.

It can be confirmed that referring to FIG 3A, the solution in the tube is yellow, and referring to FIG 3B, the solution in the tube is red. The test tube 130 and the control tube 140 may contain reagents whose color changes depending on pH, and in the example illustrated in FIG 3, "yellow" may refer to "positive" in which a nucleic acid to be detected is contained, "red" may refer to "negative" in which a nucleic acid to be detected is not contained.

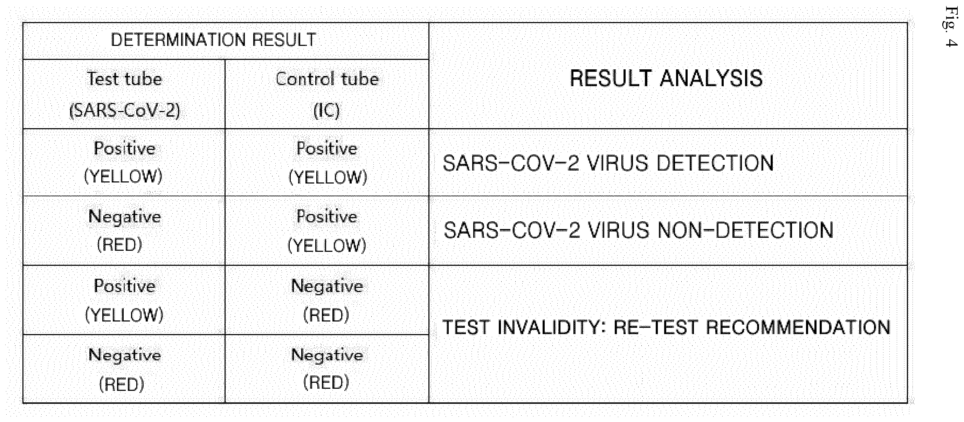

FIG 4 is an exemplary table of interpretation of results according to the color of the tube.

Referring to the table of FIG 4, when the control tube is measured to be yellow, a sample is extracted well, and an LAMP reaction is also performed well, which means that the measurement result is reliable. On the other hand, when the control tube is measured to be red, it means that the sample is not extracted well or the LAMP reaction is performed well, which means that the measurement result is unreliable. In this case, since the test is invalid, a re-test may be recommended.

On the other hand, when the control tube is yellow and the test tube is yellow, the Corona 19 virus has been detected. That is, it may be determined that the subject is infected with the COVID-19 virus. On the other hand, when the control tube is yellow and the test tube is red, the COVID-19 virus has not been detected. That is, it may be determined that the subject is not infected with the COVID-19 virus. It is obvious that a color may variously change according to a type of reagents included in the test tube 130 and the control tube 140.

A wireless connection between the measuring instrument 200 and the communication terminal 300 is required in order to measure the presence of infection using the diagnostic system according to the present specification. However, it is described under the assumption that the example described with reference to FIGS. 1 to 4 is a case in which the measuring instrument 200 and the communication terminal 300 are present in a 1:1 ratio. Depending on the use environment, a plurality of measuring instruments 200 may be connected to one communication terminal 300 and used.

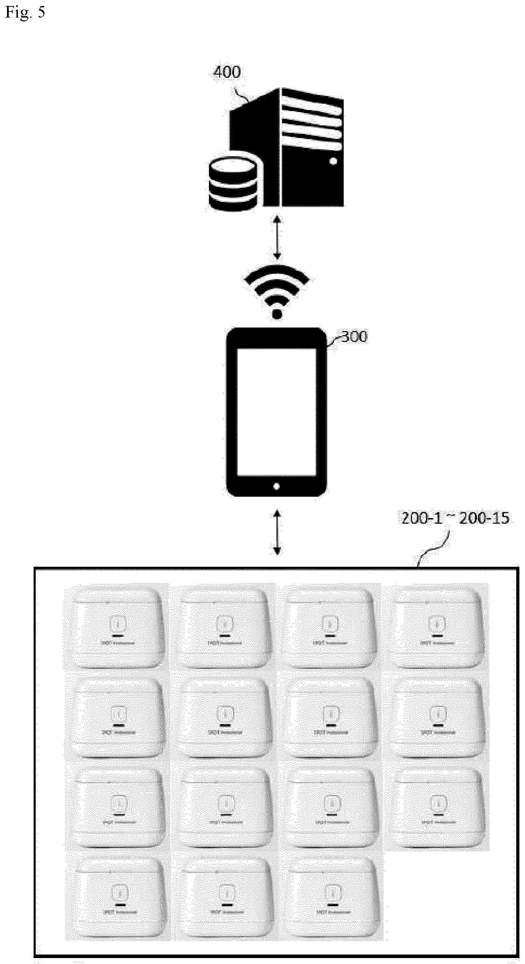

FIG 5 is a schematic configuration diagram of a communication terminal connected to a plurality of measuring instruments and a server.

Referring to FIG 5, it can be confirmed that 15 measuring instruments 200-1 to 200-15 are connected to one communication terminal 300. In addition, the communication terminal 300 may be connected to the server 400 through a communication network to transmit and receive data to and from the server 400. The number of measuring instruments connected to one communication terminal 300 may vary, and for example, in the case of a Bluetooth communication module, 1:7 or 1:15 connection is possible depending on the version. Accordingly, the number of measuring instruments illustrated in FIG 5 corresponds to one example.

The case in which it is necessary to connect a plurality of measuring instruments to one communication terminal 300 may be a case in which a small number of managers manage a plurality of examiners such as a hospital, a laboratory, a company, and a school. In this case, when the communication terminal 300 transmits the test result made by the plurality of measuring instruments 200 connected thereto to the server 400, the amount of transmitted data increases by the number of connected measuring instruments.

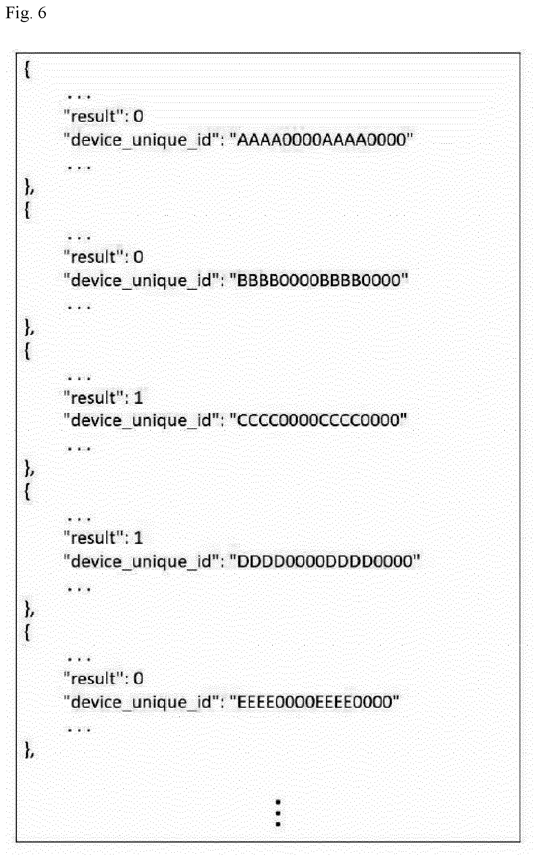

FIG 6 is a partial example of data for a plurality of measurement results.

Referring to FIG 6, the "result" is information indicating whether the nucleic acid to be detected is included in a sample measured by each measuring instrument, that is, "positive" or "negative." A value of "0" is represents negative, and a value of "1" represents positive. In this way, when the test is made by the measuring instruments, identification information ("device_unique_id") of each measuring instrument and a test result value of each measuring instrument need to be distinguished and transmitted. In this process, as the number of measuring instruments connected to one communication terminal increases and the number of communication terminals that transmit data to the server increases, data that the server needs to receive and store will increase and the communication resources used in the transmission process will increase. In addition, it is clear that the amount of data will increase exponentially as the number of tests is repeated.

As such, there is a need for a method of efficiently transmitting measurement data to a server in a situation in which one communication terminal 300 and the plurality of measuring instruments 200 are connected to each other. Hereinafter, a method of transmitting measurement result data and apparatus according to the present specification will be described with reference to the accompanying drawings.

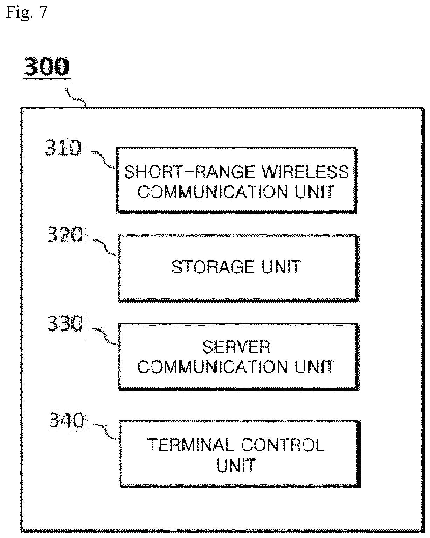

FIG 7 is a block diagram schematically illustrating a configuration of an apparatus for transmitting measurement result data according to the present specification.

Referring to FIG 7, the apparatus 300 for transmitting measurement result data according to the present specification may include a short-range wireless communication unit 310, a storage unit 320, a server communication unit 330, and a terminal control unit 340.

The short-range wireless communication unit 310 may transmit and receive data to and from the measuring instruments 200. The storage unit 320 may store data received through the short-range wireless communication unit 310. The server communication unit 330 may transmit and receive data to and from the server according to a control signal of the terminal control unit 340. The apparatus 300 for transmitting measurement result data according to the present specification may basically perform a role of the communication terminal 300 illustrated in FIG 1. Therefore, in the present specification, the apparatus 300 for transmitting measurement result data and the communication terminal 300 are described using the same reference numerals.

The apparatus 300 for transmitting measurement result data may include a terminal control unit 340 for executing the method of transmitting measurement result data according to the present specification. The terminal control unit 340 may include processors, application-specific integrated circuits (ASICs), other chipsets, logic circuits, registers, communication modems, data processing devices, etc., known in the art to which the present invention pertains for performing calculations and various control logics. In addition, when the above-described control logic is implemented in software, the terminal control unit 340 may be implemented as a set of program modules. In this case, the program module may be stored in the storage unit 320 and executed by the processor.

Hereinafter, the method of transmitting measurement result data according to the present specification will be described.

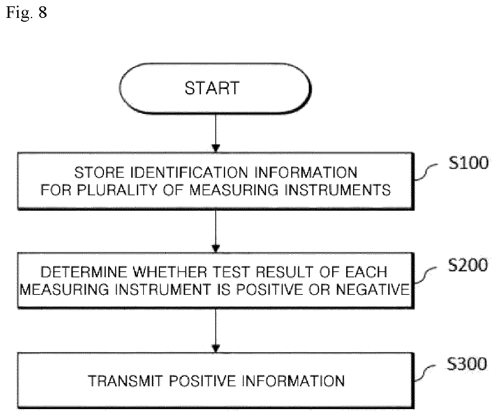

FIG 8 is a schematic flowchart of the method of transmitting measurement result data according to the present specification.

Referring to FIG 8, in the method of transmitting measurement result data according to the present specification, in operation S 100, the processor may first store identification information on measuring instruments connected through short-range wireless communication. The identification information on the measuring instrument may be unique information assigned to each measuring instrument during manufacture. In the next operation S200, the processor may determine whether the nucleic acid to be detected is included in a sample being measured by each measuring instrument (hereinafter, "positive or negative") using measured values output from each measuring instrument. In the next operation S300, the processor may transmit the identification information on the measuring instrument having a sample determined to be positive among the plurality of measuring instruments to the server. In the method of transmitting measurement result data according to the present specification, the information on the measuring instrument having a sample determined to be positive is transmitted for efficient data transmission.

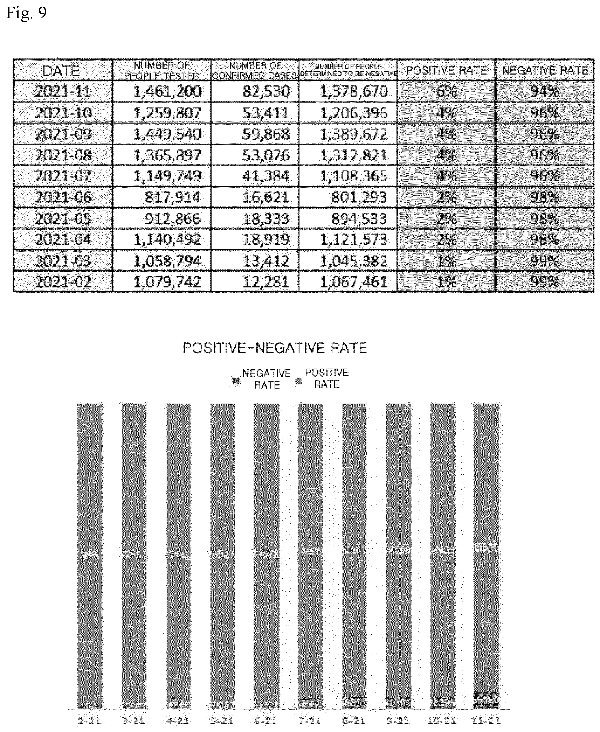

FIG 9 is a diagram illustrating data on results of a Korean coronavirus test from February to November 2021.

Referring to FIG 9, it can be confirmed that, as a result of the coronavirus test, the negative rate is overwhelmingly higher, with an average of about 97% or more. Accordingly, the present applicant has noticed that it is possible to increase the efficiency in the data transmission process when transmitting positive-related data rather than transmitting all test results and considering the remaining items as negative. That is, even if the server knows only the identification information on the measuring instrument having a sample determined to be positive among the identification information for the plurality of measuring instruments, the result of the remaining measuring instruments may be regarded as negative. To this end, the apparatus 300 for transmitting measurement result data according to the present specification needs to transmit the information on all the measuring instruments and the information on the measuring instrument having the sample determined to be positive among the plurality of measuring instruments. In this process, various embodiments are possible depending on how the information on all measuring instruments and the information on the measuring instrument having the sample determined to be positive are transmitted.

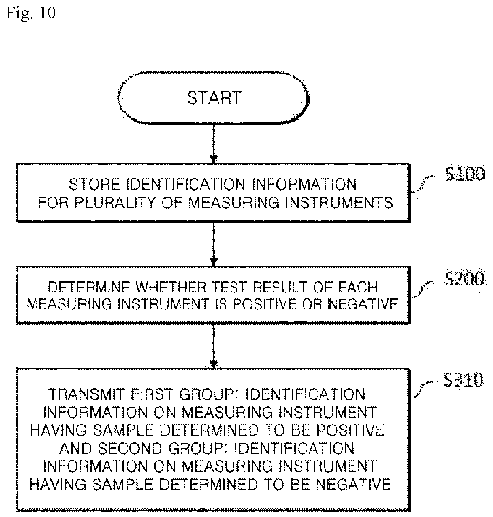

FIG 10 is a schematic flowchart of a method of transmitting measurement result data according to a first embodiment of the present specification.

Referring to FIG 10, since operations S100 and S200 are the same as those of FIG 8 above, a repetitive description thereof will be omitted. After operation S200, in operation S310, the processor transmits the identification information on a measuring instrument having a sample determined to be positive as first group information, and the processor transmits the identification information on a measuring instrument having a sample determined to be negative as second group information.

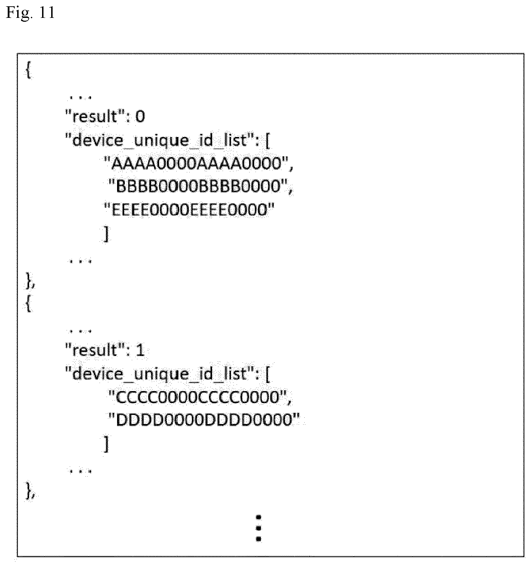

FIG 11 is an example of data transmitted according to the first embodiment of the present specification.

Referring to FIG 11, it may be seen that a group is formed by bundling the identification information on the measuring instruments having samples determined to be negative, and a group is formed by bundling the identification information on the measuring instruments having samples determined to be positive. As such, when information on measuring instruments having the same test results are grouped and transmitted, the amount of data to be transmitted and received may be reduced compared to distinguishing and transmitting the test results of each measuring instrument. The first embodiment is an embodiment in which the entire list of a plurality of measuring instruments and the test results of each measuring instrument are transmitted together in operation S310.

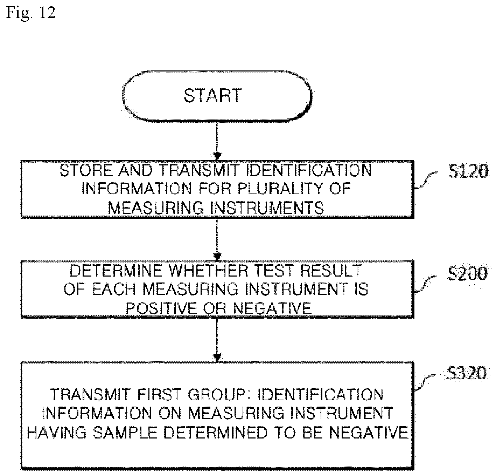

FIG 12 is a flowchart of a method of transmitting measurement result data according to a second embodiment of the present specification.

Referring to FIG 12, it can be confirmed that operation S100 changes to operation S120 and operation S300 changes to operation S320 when compared to FIG 8. In operation S120, the processor may store the identification information for the plurality of measuring instruments connected through short-range wireless communication, and transmit the identification information for the plurality of measuring instruments to the server. Since the next operation S200 is the same as that of FIG 8 above, a repetitive description thereof will be omitted. After operation S200, in operation S320, the processor may transmit the identification information on the measuring instrument having a sample determined to be positive as the first group information.

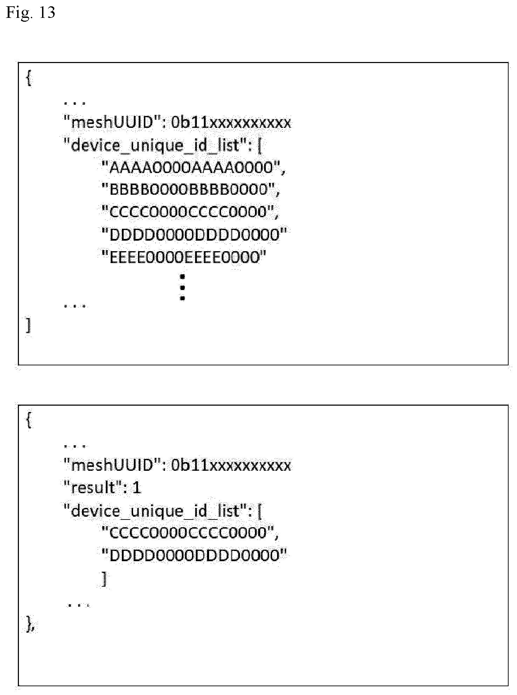

FIG 13 is an example of data transmitted according to the second embodiment of the present specification.

Referring to FIG 13, the identification information for a plurality of measuring instruments transmitted by the processor in operation S120 may be checked at the upper portion of the FIG 13. In this case, the transmitted measuring instrument is list information on all the measuring instruments connected to the communication terminal or the measuring instruments that perform a test. The identification information on the measuring instrument having a sample determined to be positive transmitted by the processor in operation S320 may be confirmed at the lower portion of FIG 13. Since the server knows the information on all the measuring instruments in advance, it may be considered that the test result of the remaining measuring instruments other than the information on the measuring instrument received in operation S320 is negative. As such, when the results for the measuring instruments having samples determined to be negative among the test results are not transmitted, the amount of data to be transmitted/received may be reduced compared to distinguishing and transmitting the test results of each measuring instrument. The second embodiment is an embodiment in which the entire list of a plurality of measuring instruments is first transmitted in operation S120, and the test results of the measuring instrument shaving samples determined to be positive in operation S320 is transmitted.

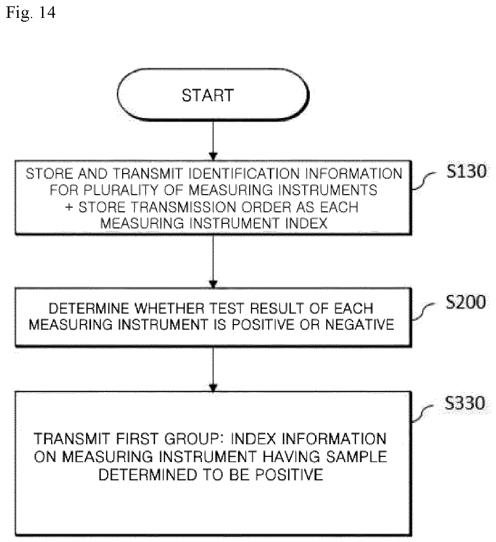

FIG 14 is a flowchart of a method of transmitting measurement result data according to a third embodiment of the present specification.

Referring to FIG 14, it can be confirmed that operation S100 changes to operation S130 and operation S300 changes to operation S330 when compared to FIG 8. In operation S120, the processor may store the identification information for the plurality of measuring instruments connected through short-range wireless communication, and transmit the identification information on the measuring instruments to the server. When transmitting the identification information for the plurality of measuring instruments to the server, the processor may further store an order in which the identification information on each measuring instrument is written as index information on each measuring instrument. Since the next operation S200 is the same as that of FIG 8 above, a repetitive description thereof will be omitted. After operation S200, in operation S330, the processor may transmit the index information on each measuring instrument having a sample determined to be positive by the processor as the first group information.

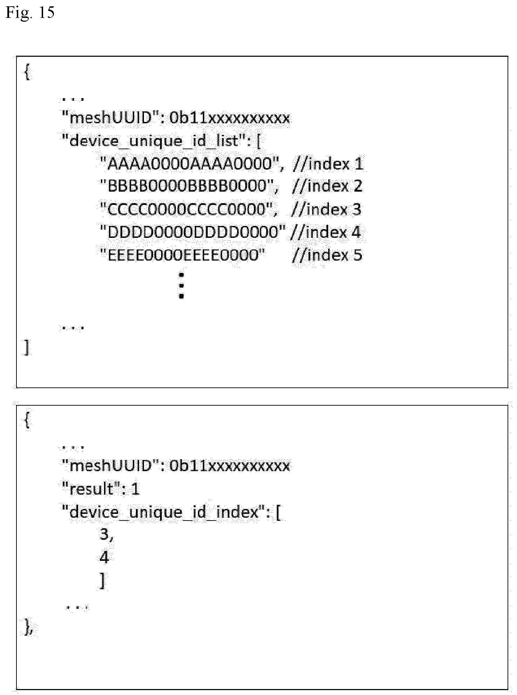

FIG 15 is an example of data transmitted according to the third embodiment of the present specification.

Referring to FIG 15, the identification information for the plurality of measuring instruments transmitted by the processor in operation S130 may be confirmed at the upper portion of the FIG 15. In this case, the order in which the transmitted identification information on the measuring instrument is written may be the index information on each measuring instrument. When the server also receives data, the index information on each measuring instrument may be provided in the order in which the identification information on the measuring instrument in the received data is written. The index information on the measuring instrument having a sample determined to be positive transmitted by the processor in operation S330 may be confirmed at the lower portion of FIG 15. Since the server knows the information on all the measuring instruments and their order, it is possible to distinguish which measuring instrument has a positive test result only with the index information on the measuring instrument received in operation S330. The server may consider that the remaining measuring instruments have a negative test result. As such, it is possible to further reduce the amount of data when transmitting the test results using the described order when transmitting the identification number of the measuring instrument. Furthermore, as in the second embodiment, since the results for the measuring instrument having a sample determined to be negative among the test results are not transmitted, it is possible to reduce the amount of data to be transmitted/received compared to distinguishing and transmitting the test results of each measuring instrument. The third embodiment is an embodiment in which the entire list of a plurality of measuring instruments is first transmitted in operation S130, and the index of the measuring instrument having a sample determined to be positive in operation S330 is transmitted.

Meanwhile, the communication terminal 300 may transmit the time series data of each measuring instrument together when transmitting the data on the test result to the server. In the present specification, the "time-series data" refers to a measured value output at each preset time in the measurement process of each measuring instrument. The measuring instrument 200 may transmit the value output from the spectrum sensor as the measured value to the communication terminal 300. In this case, the measuring instrument 200 may transmit the measured value only once after nucleic acid amplification is completed and the nucleic acid has sufficiently cooled down, but the measured value may be repeatedly transmitted at predetermined intervals for a predetermined time from the moment the amplification starts to the end of the amplification and until the nucleic acid has cooled down.

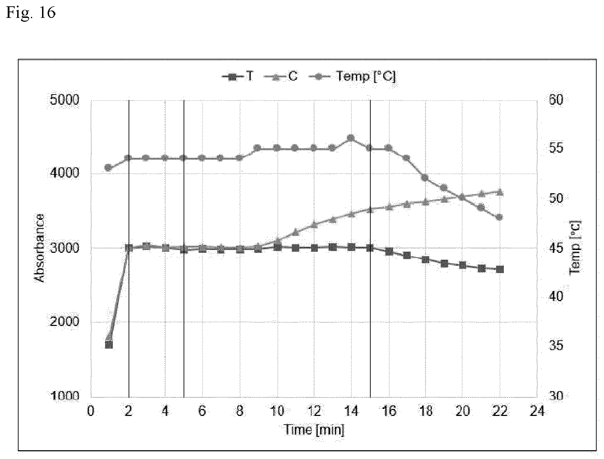

FIG 16 is an example of a change in absorbance of the control tube and the test tube over time for a sample determined to be negative.

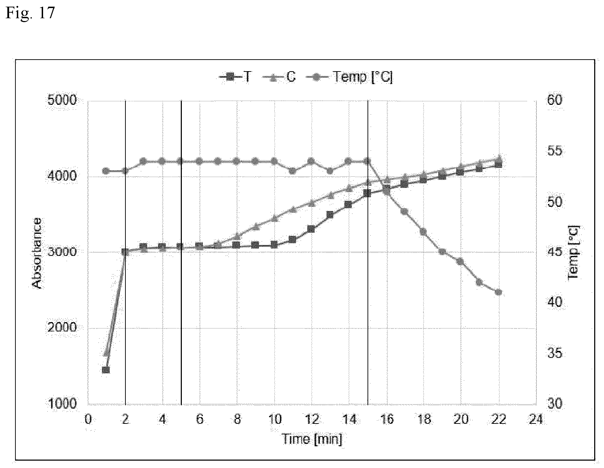

FIG 17 is an example of the change in absorbance of the control tube and the test tube over time for a sample determined to be positive.

Referring to FIGS. 16 and 17 together, it can be confirmed that the absorbance (measured value) of the sample tube determined to be negative and the absorbance (measured value) of the sample tube determined to be positive show different aspects. It can be confirmed that, in the case of negative, there is little change in the measured value, but in the case of positive, it can be confirmed that there is a change in the measured value as the nucleic acid amplification is performed. In the case of the positive, it may be necessary to analyze not only the result value, but also what kind of change process was performed to determine whether the result was positive.

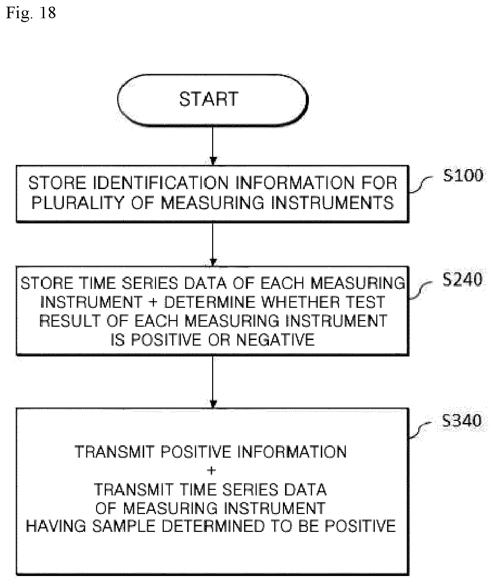

FIG 18 is a flowchart of a method of transmitting measurement result data according to a fourth embodiment of the present specification.

Referring to FIG 18, it can be confirmed that operation S200 changes to operation S240 and operation S300 changes to operation S340 when compared to FIG 8. Since the operation S 100 is the same as that of FIG 8 above, a repetitive description thereof will be omitted. In the next operation S240, the processor may store the measured value (hereinafter, 'time series data') output at each preset time in the measurement process in each measuring instrument. The processor may determine whether the test result of each measuring instrument is positive or negative. In the next step S340, the processor may transmit the time series data of the measuring instrument having the sample determined to be positive to the server together. In the fourth embodiment, any one of the first to third embodiments described above may be applied to information on all the measuring instruments and information on the measuring instrument having a sample determined to be positive.

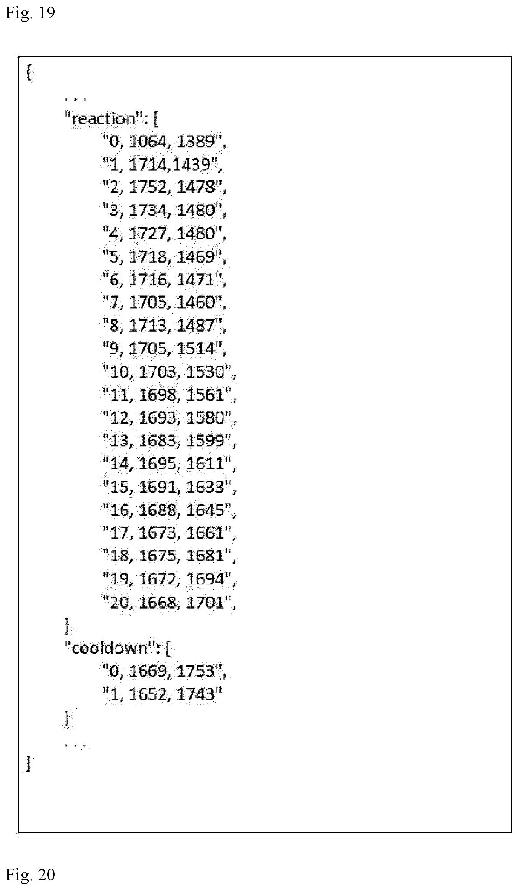

FIG 19 is an example of the time series data.

Referring to FIG 19, it is possible to check the measured values output from the test tube and the control tube during the reaction (heating) and the measured values output from the test tube and the control tube during the cooldown (after heating is finished). It is possible to further reduce the burden of use of communication resources by transmitting only the time series data of the measuring instrument having a sample determined to be positive rather than transmitting all the time series data output from all the measuring instruments.

Meanwhile, as illustrated in FIG 19, it can be confirmed that the amount of time series data output from one measurement is not small. For such time series data, as the number of measuring instruments for which the test result is determined to be positive increases, the amount of data will suddenly increase. Therefore, it is necessary to consider a method of reducing the amount of data included in the time series data.

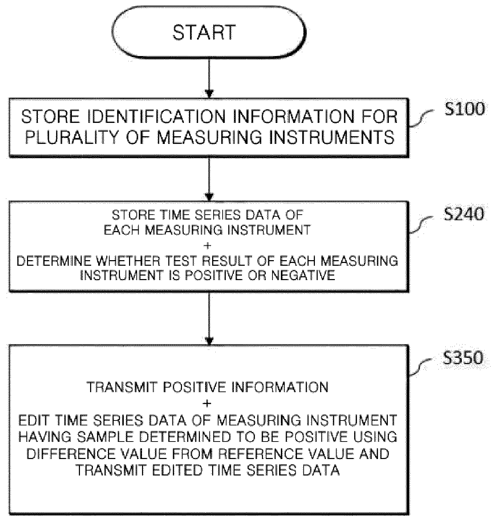

FIG 20 is a flowchart of a method of transmitting measurement result data according to a fifth embodiment of the present specification.

Referring to FIG 20, it can be confirmed that step S340 changes to step S350 when compared with FIG 18 above. Since the operations S 100 and S240 are the same as those of FIG 18 above, a repetitive description thereof will be omitted. In operation S350, the processor may edit the time series data using a difference value between a preset reference value and each measured value and transmit the edited time series data to the server. In the fifth embodiment, any one of the first to third embodiments described above may be applied to the information on all the measuring instruments and the information on the measuring instrument having a sample determined to be positive.

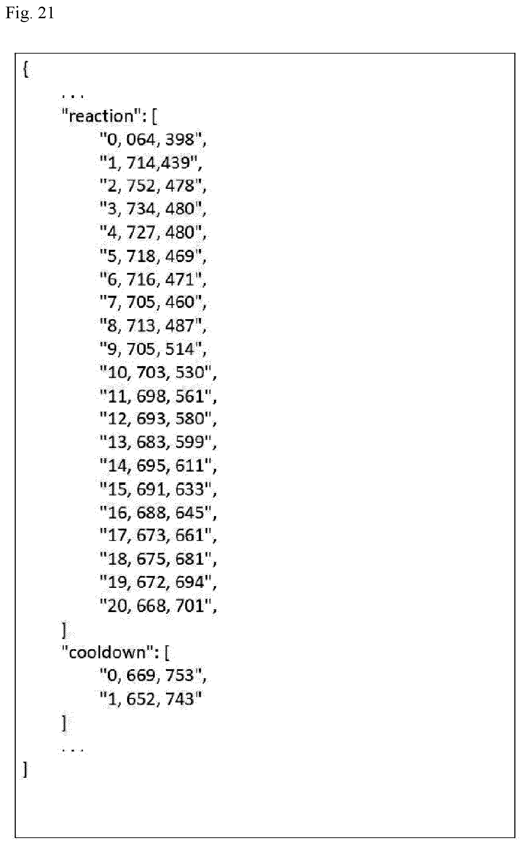

FIG 21 is an example of editing the time series data using the preset reference value.

Referring to FIG 21, as compared with the example illustrated in FIG 19, it can be confirmed that the measured value is a 3-digit number instead of a 4-digit number. The solution contained in the test tube and the sample tube has a basic color before heating (before amplification), so there is a possibility that the measured value output from the spectrum sensor will also output more than a default value. Accordingly, as in the example illustrated in FIG 19, when the measured value is a 4-digit value and a first digit does not change, the default value may be set to "1000" in advance and edited using a difference value between each measured value. In this case, the preset default value may be shared in advance with the communication terminal 300 and the server 400, and an original value may be restored after the server 400 receives the time series data. The edited time series data and default value illustrated in FIG 21 are only examples, and do not limit the method of transmitting measurement result data described in the present specification.

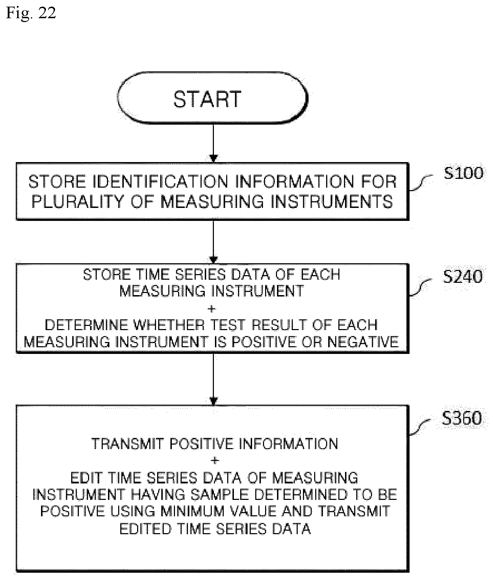

FIG 22 is a flowchart of a method of transmitting measurement result data according to a sixth embodiment of the present specification.

Referring to FIG 22, it can be confirmed that step S340 changes to step S350 when compared with FIG 18 above. Since the operations S100 and S240 are the same as those of FIG 18 above, a repetitive description thereof will be omitted. In operation S360, the processor may edit the time series data using a difference value between minimum value information in the time series data and each measured value and transmit the edited time series data to the server. In the sixth embodiment, any one of the first to third embodiments described above may be applied to the information on all the measuring instruments and the information on the measuring instrument having a sample determined to be positive.

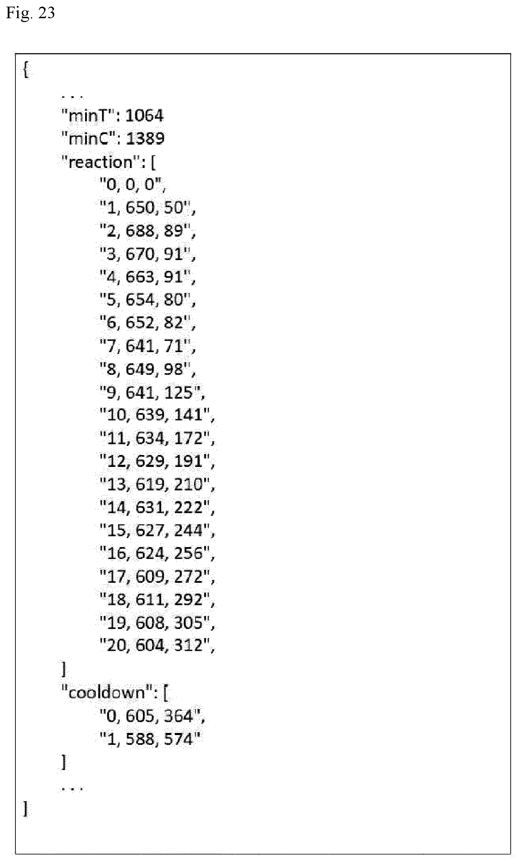

FIG 23 is an example of editing the time series data using the minimum value in the time series data.

Referring to FIG 23, it can be confirmed that a minimum value (minT) "1064" of the test tube and a minimum value (minC) "1389" of the control tube are added when compared with the example illustrated in FIG 19. It can be confirmed that the remaining measured values are edited using a difference value from the minimum value. After receiving the time series data, the server 400 may restore the original value using the minimum value. The edited time series data illustrated in FIG 23 is only an example of using the value described in FIG 21, and does not limit the method of transmitting measurement result data described in the present specification.

Meanwhile, although the time series data transmitted in the fifth and sixth embodiments according to the present specification has an advantage of including accurate measured values, the amount of data is not small. Accordingly, there may be a need for a method for more simply transmitting time series data.

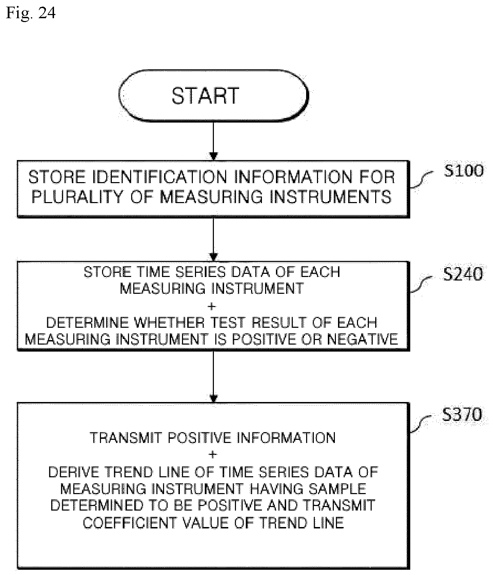

FIG 24 is a flowchart of a method of transmitting measurement result data according to a seventh embodiment of the present specification.

Referring to FIG 24, it can be confirmed that step S340 changes to step S370 when compared with FIG 18 above. Since the operations S 100 and S240 are the same as those of FIG 18 above, a repetitive description thereof will be omitted. In operation S370, the processor may derive the trend line of the time series data and transmit a coefficient value of the derived trend line to the server. In the seventh embodiment, any one of the first to third embodiments described above may be applied to the information on all the measuring instruments and the information on the measuring instrument having a sample determined to be positive.

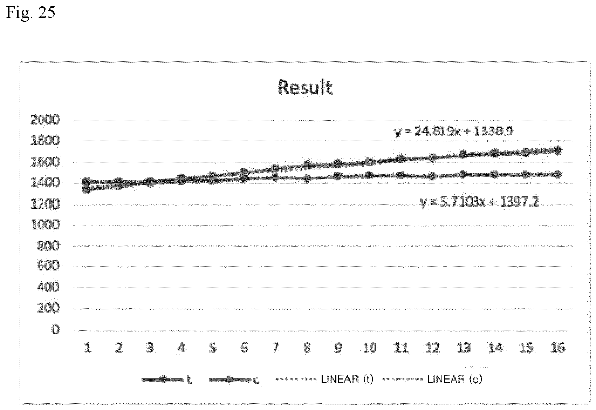

FIG 25 is an exemplary diagram of the trend line of the time series data.

Referring to FIG 25, the trend line (dotted line) derived using the measured values measured in the test tube and the control tube may be confirmed. According to an embodiment of the present specification, the trend line may be derived through a least squares linear equation.

<Equation>

$$a \sum x^2 + b \sum x = \sum xy$$

$$a \sum x + bn = \sum y$$

As the least squares linear equation can be seen in the above equation, values for coefficient values a and b are derived. The processor may transmit the coefficient value to the server to reduce the total amount of data to be transmitted. Meanwhile, when transmitting the coefficient value, the processor may also transmit a range of valid data.

According to an embodiment of the present specification, the processor may calculate error values between each measured value included in the time series data and the derived trend line and transmit the coefficient value of the derived trend line to the server when the number of the error values exceeding a preset error range is less than or equal to a preset reference number.

Meanwhile, in the first embodiment and the seventh embodiment, only the case in which the test result is "positive" or "negative" is described for convenience of explanation. However, in the actual test process, there may be cases where the test result is "invalid" or "error." "Invalid" corresponds to the case in which the nucleic acid amplification reaction did not occur in the control tube since the amount of collected sample is insufficient. The "error" corresponds to a case in which the test process is not completely completed, such as when the lid of the measuring instrument is opened during the test process. The method of transmitting measurement result data according to the present specification may additionally transmit unique identification information of the measuring instrument in which the "invalid" or "error" occurs as well as the "positive" information when the "invalid" or "error" occurs.

In order for the computer to read the program and execute the methods implemented as a program, the computer program may include code coded in a computer language such as C/C++, C#, JAVA, Python, machine language, and the like that the processor (CPU) of the computer can read through a device interface of the computer. Such code may include functional code related to functions defining functions necessary for executing the methods, or the like, and include execution procedure-related control code necessary for the processor of the computer to execute the functions according to a predetermined procedure. In addition, such code may further include memory reference-related code for which location (address, house number) of the internal or external memory of the computer additional information or media necessary for the processor of the computer to execute the functions should be referenced. In addition, when the processor of the computer needs to communicate with any other computers, servers, or the like located remotely in order to execute the above functions, the code may further include communication-related code for how to communicate with any other computers, servers, or the like located remotely using a communication module of the computer, how to transmit/receive any information or media during communication, or the like.

The storage medium is not a medium that stores data therein for a short moment, such as a register, a cache, a memory, or the like, but means a medium that semi-permanently stores data therein and is readable by a device. Specifically, examples of the storage medium include, but are not limited to, a ROM, a RAM, a CD-ROM, a magnetic tape, a floppy disk, an optical data storage device, or the like. That is, the program may be stored in various recording media on various servers accessible by the computer or in various recording media on the computer of the user. In addition, the medium may be distributed in a computer system connected by a network, and store computer-readable code in a distributed manner.

According to the present disclosure, it is possible to transmit data made by a plurality of measuring instruments with a small capacity, thereby saving communication resources.

The effects of the present invention are not limited to the above-mentioned effect, and other effects that are not mentioned may be obviously understood by those skilled in the art from the above detailed description.

Although exemplary embodiments of the present specification has been described with reference to the accompanying drawings, those skilled in the art to which the present specification belongs will appreciate that various modifications and alterations may be made without departing from the essential feature of the present invention. Therefore, it is to be understood that exemplary embodiments described above are illustrative rather than being restrictive in all aspects.

**Claims**

1. A method of transmitting measurement result data, comprising operations of:

   (a) storing, by a processor, identification information for a plurality of measuring instruments connected through short-range wireless communication;
   (b) determining, by the processor, whether a nucleic acid to be detected is included in a sample being measured by each measuring instrument (hereinafter, "positive or negative") using measured values output from each measuring instrument; and
   (c) transmitting, by the processor, identification information on a measuring instrument having a sample determined to be positive among the plurality of measuring instruments to a server.

2. The method of claim 1, wherein the operation (c) includes:

   transmitting, by the processor, the identification information on the measuring instrument having the sample determined to be positive as first group information, and
   transmitting, by the processor, identification information on a measuring instrument having a sample determined to be negative as second group information.

3. The method of claim 1 or 2, wherein the operation (a) includes transmitting, by the processor, the identification information for the plurality of measuring instruments to the server, and
the operation (c) includes transmitting, by the processor, the identification information on the measuring instrument having the sample determined to be positive as first group information.

4. The method of any one of claims 1 to 3, wherein the operation (a) includes further storing an order in which the identification information on each measuring instrument is written as index information on each measuring instrument when the processor transmits the identification information for the plurality of measuring instruments to the server, and
the operation (c) includes transmitting, by the processor, the index information on the measuring instrument having the sample determined to be positive as the first group information.

5. The method of any one of claims 1 to 4, wherein the operation (b) includes further storing, by the processor, a measured value as a time series data output at each preset time in the measurement process in each measuring instrument, and
the operation (c) includes further transmitting, by the processor, the time series data of the measuring instrument having the sample determined to be positive to the server.

6. The method of claim 5, wherein the operation (c) includes editing, by the processor, the time series data using a difference value between a preset reference value and each measured value and transmitting the edited time series data to the server.

7. The method of claim 5, wherein the operation (c) includes editing, by the processor, the time series data using a difference value between a minimum value in the time series data and each measured value and transmitting the edited time series data to the server.

8. The method of claim 5, wherein the operation (c) includes deriving, by the processor, a trend line of the time series data and transmitting the derived time series data to the server.

9. The method of claim 8, wherein the operation (c) includes calculating, by the processor, error values between each measured value included in the time series data and the derived trend line and transmitting a coefficient value of the derived trend line to the server when the number of the error values exceeding a preset error range is less than or equal to a preset reference number.

10. A non-transitory computer readable recording medium recording a computer program for performing operations of the measurement result data transmitting method according to any one of claims 1 to 9 in a computer and recorded on a computer-readable recording medium.

11. An apparatus for transmitting measurement result data, comprising:

a short-range wireless communication unit configured to transmit and receive data to and from a plurality of measuring instruments;
a storage unit configured to store the data received through the short-range wireless communication unit;
a server communication unit configured to transmit and receive data to and from a server; and
a control unit configured to control a server communication unit to store identification information for the plurality of measuring instruments connected through the short-range wireless communication unit in the storage unit, use measured values output from each measuring instrument to determine whether a nucleic acid to be detected is included in a sample being measured by each measuring instrument (hereinafter referred to as "positive or negative"), and transmit identification information on a measuring instrument having a sample determined to be positive among the measuring instruments to the server.

12. The apparatus of claim 11, wherein the control unit transmits the identification information on the measuring instrument having the sample determined to be positive as first group information, and
allows the processor to transmit identification information on a measuring instrument having a sample determined to be negative as second group information.

13. The apparatus of claim 11 or 12, wherein the control unit transmits the identification information for the plurality of measuring instruments to the server, and transmits the identification information on the measuring instrument having the sample determined to be positive as first group information.

14. The apparatus of any one of claims 11 to 13, wherein, when the processor transmits the identification information for the plurality of measuring instruments to the server, the control unit further stores an order, in which the identification information on each measuring instrument is written, as index information on each measuring instrument, and transmits the index information on the measuring instrument having the sample determined to be positive as the first group information.

15. The apparatus of any one of claims 11 to 14, wherein the control unit allows the processor to further store the measured value as a time series data output at each preset time in the measurement process in each measuring instrument, and further store the time series data of the measuring instrument having the sample determined to be positive to the server.

16. The apparatus of claim 15, wherein the control unit edits the time series data using a difference value between a preset reference value and each measured value and transmits the edited time series data to the server.

17. The apparatus of claim 15, wherein the control unit edits the time series data using a difference value between a minimum value in the time series data and each measured value and transmits the edited time series data to the server.

18. The apparatus of claim 15, wherein the control unit derives a trend line of the time series data and transmits a coefficient value of the derived trend line to the server.

19. The apparatus of claim 18, wherein the control unit calculates error values between each measured value included in the time series data and the derived trend line and transmits the coefficient value of the derived trend line to the server when the number of the error values exceeding a preset error range is less than or equal to a preset reference number.

Fig. 1

**10**

100

110

120

130

140

200

300

Fig. 2

Fig. 3

(a)                    (b)

Fig. 4

EP 4 350 702 A1

| DETERMINATION RESULT | | RESULT ANALYSIS |
|---|---|---|
| Test tube (SARS-CoV-2) | Control tube (IC) | |
| Positive (YELLOW) | Positive (YELLOW) | SARS-COV-2 VIRUS DETECTION |
| Negative (RED) | Positive (YELLOW) | SARS-COV-2 VIRUS NON-DETECTION |
| Positive (YELLOW) | Negative (RED) | TEST INVALIDITY: RE-TEST RECOMMENDATION |
| Negative (RED) | Negative (RED) | |

Fig. 5

Fig. 6

```
{
    . . .
    "result": 0
    "device_unique_id": "AAAA0000AAAA0000"
    . . .
},
{
    . . .
    "result": 0
    "device_unique_id": "BBBB0000BBBB0000"
    . . .
},
{
    . . .
    "result": 1
    "device_unique_id": "CCCC0000CCCC0000"
    . . .
},
{
    . . .
    "result": 1
    "device_unique_id": "DDDD0000DDDD0000"
    . . .
},
{
    . . .
    "result": 0
    "device_unique_id": "EEEE0000EEEE0000"
    . . .
},
                    .
                    .
                    .
```

Fig. 7

**300**

310 — SHORT–RANGE WIRELESS COMMUNICATION UNIT

320 — STORAGE UNIT

330 — SERVER COMMUNICATION UNIT

340 — TERMINAL CONTROL UNIT

Fig. 8

Fig. 9

| DATE | NUMBER OF PEOPLE TESTED | NUMBER OF CONFIRMED CASES | NUMBER OF PEOPLE DETERMINED TO BE NEGATIVE | POSITIVE RATE | NEGATIVE RATE |
|---|---|---|---|---|---|
| 2021-11 | 1,461,200 | 82,530 | 1,378,670 | 6% | 94% |
| 2021-10 | 1,259,807 | 53,411 | 1,206,396 | 4% | 96% |
| 2021-09 | 1,449,540 | 59,868 | 1,389,672 | 4% | 96% |
| 2021-08 | 1,365,897 | 53,076 | 1,312,821 | 4% | 96% |
| 2021-07 | 1,149,749 | 41,384 | 1,108,365 | 4% | 96% |
| 2021-06 | 817,914 | 16,621 | 801,293 | 2% | 98% |
| 2021-05 | 912,866 | 18,333 | 894,533 | 2% | 98% |
| 2021-04 | 1,140,492 | 18,919 | 1,121,573 | 2% | 98% |
| 2021-03 | 1,058,794 | 13,412 | 1,045,382 | 1% | 99% |
| 2021-02 | 1,079,742 | 12,281 | 1,067,461 | 1% | 99% |

POSITIVE-NEGATIVE RATE

NEGATIVE RATE    POSITIVE RATE

Fig. 10

```
                    ┌─────────────────┐
                    │      START      │
                    └─────────────────┘
                             │
                             ▼
         ┌──────────────────────────────────────┐
         │   STORE IDENTIFICATION INFORMATION    │ ─── S100
         │ FOR PLURALITY OF MEASURING INSTRUMENTS │
         └──────────────────────────────────────┘
                             │
                             ▼
         ┌──────────────────────────────────────┐
         │  DETERMINE WHETHER TEST RESULT OF EACH │ ─── S200
         │ MEASURING INSTRUMENT IS POSITIVE OR NEGATIVE │
         └──────────────────────────────────────┘
                             │
                             ▼
         ┌──────────────────────────────────────┐
         │   TRANSMIT FIRST GROUP: IDENTIFICATION │ ─── S310
         │   INFORMATION ON MEASURING INSTRUMENT  │
         │ HAVING SAMPLE DETERMINED TO BE POSITIVE │
         │       AND SECOND GROUP: IDENTIFICATION  │
         │   INFORMATION ON MEASURING INSTRUMENT   │
         │ HAVING SAMPLE DETERMINED TO BE NEGATIVE │
         └──────────────────────────────────────┘
```

Fig. 11

```
{
    . . .
    "result": 0
    "device_unique_id_list": [
        "AAAA0000AAAA0000",
        "BBBB0000BBBB0000",
        "EEEE0000EEEE0000"
        ]
    . . .
},
{
    . . .
    "result": 1
    "device_unique_id_list": [
        "CCCC0000CCCC0000",
        "DDDD0000DDDD0000"
        ]
    . . .
},
                            :
                            :
```

Fig. 12

START

STORE AND TRANSMIT IDENTIFICATION INFORMATION FOR PLURALITY OF MEASURING INSTRUMENTS — S120

DETERMINE WHETHER TEST RESULT OF EACH MEASURING INSTRUMENT IS POSITIVE OR NEGATIVE — S200

TRANSMIT FIRST GROUP: IDENTIFICATION INFORMATION ON MEASURING INSTRUMENT HAVING SAMPLE DETERMINED TO BE NEGATIVE — S320

Fig. 13

```
{
    . . .
    "meshUUID": 0b11xxxxxxxxxx
    "device_unique_id_list": [
        "AAAA0000AAAA0000",
        "BBBB0000BBBB0000",
        "CCCC0000CCCC0000",
        "DDDD0000DDDD0000"
        "EEEE0000EEEE0000"
                    ⋮
    . . .
]
```

```
{
    . . .
    "meshUUID": 0b11xxxxxxxxxx
    "result": 1
    "device_unique_id_list": [
        "CCCC0000CCCC0000",
        "DDDD0000DDDD0000"
        ]
    . . .
},
```

Fig. 14

```
                    ┌─────────────┐
                    │    START    │
                    └──────┬──────┘
                           │
                           ▼
┌──────────────────────────────────────────────┐    S130
│ STORE AND TRANSMIT IDENTIFICATION INFORMATION  │
│   FOR PLURALITY OF MEASURING INSTRUMENTS       │
│      + STORE TRANSMISSION ORDER AS EACH        │
│         MEASURING INSTRUMENT INDEX             │
└──────────────────────┬─────────────────────────┘
                       │
                       ▼
┌──────────────────────────────────────────────┐    S200
│     DETERMINE WHETHER TEST RESULT OF EACH      │
│  MEASURING INSTRUMENT IS POSITIVE OR NEGATIVE  │
└──────────────────────┬─────────────────────────┘
                       │
                       ▼
┌──────────────────────────────────────────────┐    S330
│                                                │
│     TRANSMIT FIRST GROUP: INDEX INFORMATION    │
│   ON MEASURING INSTRUMENT HAVING SAMPLE        │
│          DETERMINED TO BE POSITIVE             │
│                                                │
└──────────────────────────────────────────────┘
```

Fig. 15

```
{
    . . .
    "meshUUID": 0b11xxxxxxxxxx
    "device_unique_id_list": [
        "AAAA0000AAAA0000", //index 1
        "BBBB0000BBBB0000", //index 2
        "CCCC0000CCCC0000", //index 3
        "DDDD0000DDDD0000" //index 4
        "EEEE0000EEEE0000"    //index 5
                  :
                  :
    . . .
]
```

```
{
    . . .
    "meshUUID": 0b11xxxxxxxxxx
    "result": 1
    "device_unique_id_index": [
        3,
        4
        ]
     . . .
},
```

Fig. 16

Fig. 17

Fig. 18

START

↓

STORE IDENTIFICATION INFORMATION FOR
PLURALITY OF MEASURING INSTRUMENTS — S100

↓

STORE TIME SERIES DATA OF EACH MEASURING
INSTRUMENT + DETERMINE WHETHER TEST
RESULT OF EACH MEASURING INSTRUMENT
IS POSITIVE OR NEGATIVE — S240

↓

TRANSMIT POSITIVE INFORMATION
+
TRANSMIT TIME SERIES DATA
OF MEASURING INSTRUMENT
HAVING SAMPLE DETERMINED TO BE POSITIVE — S340

Fig. 19

```
{
    . . .
    "reaction": [
        "0, 1064, 1389",
        "1, 1714,1439",
        "2, 1752, 1478",
        "3, 1734, 1480",
        "4, 1727, 1480",
        "5, 1718, 1469",
        "6, 1716, 1471",
        "7, 1705, 1460",
        "8, 1713, 1487",
        "9, 1705, 1514",
        "10, 1703, 1530",
        "11, 1698, 1561",
        "12, 1693, 1580",
        "13, 1683, 1599",
        "14, 1695, 1611",
        "15, 1691, 1633",
        "16, 1688, 1645",
        "17, 1673, 1661",
        "18, 1675, 1681",
        "19, 1672, 1694",
        "20, 1668, 1701",
    ]
    "cooldown": [
        "0, 1669, 1753",
        "1, 1652, 1743"
    ]
    . . .
]
```

Fig. 20

START

STORE IDENTIFICATION INFORMATION FOR PLURALITY OF MEASURING INSTRUMENTS — S100

STORE TIME SERIES DATA OF
EACH MEASURING INSTRUMENT
+
DETERMINE WHETHER TEST RESULT OF EACH MEASURING
INSTRUMENT IS POSITIVE OR NEGATIVE — S240

TRANSMIT POSITIVE INFORMATION
+
EDIT TIME SERIES DATA OF MEASURING INSTRUMENT
HAVING SAMPLE DETERMINED TO BE POSITIVE USING
DIFFERENCE VALUE FROM REFERENCE VALUE AND
TRANSMIT EDITED TIME SERIES DATA — S350

Fig. 21

```
{
    . . .
    "reaction": [
        "0, 064, 398",
        "1, 714,439",
        "2, 752, 478",
        "3, 734, 480",
        "4, 727, 480",
        "5, 718, 469",
        "6, 716, 471",
        "7, 705, 460",
        "8, 713, 487",
        "9, 705, 514",
        "10, 703, 530",
        "11, 698, 561",
        "12, 693, 580",
        "13, 683, 599",
        "14, 695, 611",
        "15, 691, 633",
        "16, 688, 645",
        "17, 673, 661",
        "18, 675, 681",
        "19, 672, 694",
        "20, 668, 701",
    ]
    "cooldown": [
        "0, 669, 753",
        "1, 652, 743"
    ]
    . . .
]
```

Fig. 22

```
                    ┌─────────────┐
                    │    START    │
                    └──────┬──────┘
                           │
                           ▼
┌──────────────────────────────────────────┐
│   STORE IDENTIFICATION INFORMATION FOR     │  ⌐ S100
│    PLURALITY OF MEASURING INSTRUMENTS      │
└──────────────────────┬─────────────────────┘
                       │
                       ▼
┌──────────────────────────────────────────┐
│        STORE TIME SERIES DATA OF EACH      │  ⌐ S240
│            MEASURING INSTRUMENT            │
│                     +                      │
│    DETERMINE WHETHER TEST RESULT OF EACH   │
│  MEASURING INSTRUMENT IS POSITIVE OR NEGATIVE│
└──────────────────────┬─────────────────────┘
                       │
                       ▼
┌──────────────────────────────────────────┐
│                                            │  ⌐ S360
│         TRANSMIT POSITIVE INFORMATION      │
│                     +                      │
│      EDIT TIME SERIES DATA OF MEASURING    │
│  INSTRUMENT HAVING SAMPLE DETERMINED TO BE │
│   POSITIVE USING MINIMUM VALUE AND TRANSMIT│
│            EDITED TIME SERIES DATA         │
│                                            │
└────────────────────────────────────────────┘
```

Fig. 23

```
{
    . . .
    "minT": 1064
    "minC": 1389
    "reaction": [
        "0, 0, 0",
        "1, 650, 50",
        "2, 688, 89",
        "3, 670, 91",
        "4, 663, 91",
        "5, 654, 80",
        "6, 652, 82",
        "7, 641, 71",
        "8, 649, 98",
        "9, 641, 125",
        "10, 639, 141",
        "11, 634, 172",
        "12, 629, 191",
        "13, 619, 210",
        "14, 631, 222",
        "15, 627, 244",
        "16, 624, 256",
        "17, 609, 272",
        "18, 611, 292",
        "19, 608, 305",
        "20, 604, 312",
    ]
    "cooldown": [
        "0, 605, 364",
        "1, 588, 574"
    ]
    . . .
]
```

Fig. 24

START

STORE IDENTIFICATION INFORMATION FOR
PLURALITY OF MEASURING INSTRUMENTS ⟶ S100

STORE TIME SERIES DATA OF EACH
MEASURING INSTRUMENT
+
DETERMINE WHETHER TEST RESULT OF EACH
MEASURING INSTRUMENT IS POSITIVE OR NEGATIVE ⟶ S240

TRANSMIT POSITIVE INFORMATION
+
DERIVE TREND LINE OF TIME SERIES DATA OF
MEASURING INSTRUMENT HAVING SAMPLE
DETERMINED TO BE POSITIVE AND TRANSMIT
COEFFICIENT VALUE OF TREND LINE ⟶ S370

Fig. 25

Europäisches
Patentamt

European
Patent Office

Office européen
des brevets

# EUROPEAN SEARCH REPORT

Application Number

EP 22 21 0193

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | KR 2022 0150546 A (1DROP INC [KR]) 11 November 2022 (2022-11-11) * abstract; figure 1 * * paragraph [0054] - paragraph [0055] * * paragraph [0077] * ----- | 1,10,11 | INV. G16H10/40 G01N33/487 G01N35/00 G16H40/67 H04M1/72412 H04W4/38 H04W4/80 |
| X | US 2022/011293 A1 (HUMMER MATTHEW [US] ET AL) 13 January 2022 (2022-01-13) * abstract * * paragraph [0108] * * paragraph [0199] - paragraph [0202] * * paragraph [0234] - paragraph [0236] * ----- | 1-15 | ADD. H04B5/00 H04B5/02 B01L3/00 |
| X | WO 2021/220192 A2 (STAB VIDA INVESTIG E SERVICOS EM CIENCIAS BIOLOGICAS LDA [PT]) 4 November 2021 (2021-11-04) * abstract; figure 1 * * page 23, line 15 - page 24, line 19 * * page 27, line 14 - page 29, line 8 * ----- | 1-15 | |

TECHNICAL FIELDS
SEARCHED (IPC)

G16H
G01N
B01L

The present search report has been drawn up for all claims

| Place of search | Date of completion of the search | Examiner |
|---|---|---|
| Munich | 29 August 2023 | Ricciardi, Maurizio |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 21 0193

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2023

| Patent document cited in search report | | | Publication date | Patent family member(s) | | | Publication date |
|---|---|---|---|---|---|---|---|
| KR 20220150546 | A | | 11-11-2022 | NONE | | | |
| US 2022011293 | A1 | | 13-01-2022 | US | 2022011293 | A1 | 13-01-2022 |
| | | | | US | 2022252573 | A1 | 11-08-2022 |
| WO 2021220192 | A2 | | 04-11-2021 | AU | 2021265579 | A1 | 10-11-2022 |
| | | | | BR | 112022021086 | A2 | 13-12-2022 |
| | | | | CA | 3175648 | A1 | 04-11-2021 |
| | | | | CN | 115836133 | A | 21-03-2023 |
| | | | | EP | 4142945 | A2 | 08-03-2023 |
| | | | | IL | 297281 | A | 01-12-2022 |
| | | | | JP | 2023524117 | A | 08-06-2023 |
| | | | | WO | 2021220192 | A2 | 04-11-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102229225